Europäisches Patentamt

(19) European Patent Office

Office européen des brevets

(11) Publication number : **0 259 353 B1**

(12) **EUROPEAN PATENT SPECIFICATION**

(45) Date of publication of patent specification :
**18.03.92 Bulletin 92/12**

(51) Int. Cl.$^5$ : **A61K 31/40**

(21) Application number : **87900460.4**

(22) Date of filing : **03.12.86**

(86) International application number :
**PCT/US86/02600**

(87) International publication number :
**WO 87/04927 27.08.87 Gazette 87/19**

(54) **Use of cobalt proto - and/or mesoporphyrin for the production of a medicament for suppressing the endocrine system.**

(30) Priority : **21.02.86 US 832512**
**06.11.86 US 927830**

(43) Date of publication of application :
**16.03.88 Bulletin 88/11**

(45) Publication of the grant of the patent :
**18.03.92 Bulletin 92/12**

(84) Designated Contracting States :
**AT BE CH DE FR GB IT LI LU NL SE**

(56) References cited :
**PHARMACOLOGY, vol. 34, 1986, pages 9-16 T.J. SMITH et al.: "Cobalt-Protoporphyrin suppresses thyroid and testicular hormone concentrations in rat serum: A novel action of this synthetic heme analogue"**
**MOLECULAR AND CELLULAR BIOCHEMISTRY, vol. 37, no. 1, 1981, pages 27-41 Martinus Nijhoff, The Hague, NL; G. KIKUCHI et al.: "Regulation by heme of synthesis and intracellular translocation of deltaaminolevulinate synthase in the liver"**
**PROC. NATL. ACAD. SCI., USA, vol. 79, December 1982, pages 7537-7541, T.J. SMITH et al.: "Thyroid hormone regulation of heme oxidation in the liver"**
**CHEMICAL ABSTRACTS, vol. 100, no. 7, February 13, 1984, page 96, ref. 45779k, Columbus, Ohio, US; I.V. BELOVTSKAYA: "Effect of cobalt chloride and thyroxine on key enzymes for the synthesis and degradation of heme" & FIZIOL. BIOKHIM. ONTOG. 1983, 80-1**

(56) References cited :
**XENOBIOTICA, vol. 15, no. 5. 1985, pages 407-412 G. FEUER et al.: "Association between progesterone binding and cytochrome P-450 content of hepatic microsomes in the rat treated with cobalt-haem"**
**CLINICAL RESEARCH, vol. 27, no. 3 (578A), 1979 H. IBRAHIM et al.: "Effect of estrogen and cobalt on hepatic heme in iron overload"**
**INT. J. BIOCHEM., vol. 15, no. 4, 1981, pages 479-485, Pergamon Press Ltd., GB;K.T. KITCHIN: "Regulation of rat hepatic delta-aminolevulinic acid synthetase and heme oxygenase activities: evidence for control by heme and against mediation by prosthetic iron"**
**PHARMACOLOGY, vol. 34, 1987, pages 241-249, S. Karger AG, Basel, CH R.A. GALBRAITH et al.: "Relationship of suppression of the androgenic axis by cobalt-protoporphyrin to its effects on weights loss and hepatic heme oxygenase induction"**

(73) Proprietor : **THE ROCKEFELLER UNIVERSITY**
**1230 York Avenue**
**New York, NY 10021 (US)**

(72) Inventor : **KAPPAS, Attalah**
**Griswold Road**
**Rye, NY 10580 (US)**
Inventor : **DRUMMOND, George**
**304 West 75th Street**
**New York, NY 10023 (US)**

(74) Representative : **Weinhold, Peter, Dr. et al**
**Patentanwälte Dr. V. Schmied-Kowarzik**
**Dipl.-Ing. G. Dannenberg Dr. P. Weinhold Dr. D. Gudel Dipl.-Ing. S. Schubert Dr. P. Barz**
**Siegfriedstrasse 8**
**W-8000 München 40 (DE)**

EP 0 259 353 B1

## Description

This invention is concerned with the suppressing on of the endocine system of mammals. More particularly, it is concerned with the limiting of the activity of the glands of the endocrine system to achieve desirable physiological results such as suppression of hormone production of mammals, including humans, with cobalt protoporphyrin (CoPP) and cobalt mesoporphyrin (CoMP). It is concerned also with the production of therapeutically useful isotonic compositions containing CoPP and/or CoMP. Both of these compounds are known.

Moveover, from Molecular and Cellular Biochemistry, Vol. 37, No.1, 1981, pages 27-41, Martinus Nijhoff, The Hague, NL, it is known that administration of CoPP results in the suppression of ALA synthétase.

The endocrine system is responsible for the production and distribution in mammals of hormones which influence such factors as phenotype, homeostasis, weight, size and behavior of the individual. The principal organs of the endocrine system are the hypothalamus, pituitary (anterior and posterior), parathyroid, thyroid, thymus, adrenal, pancreas and gonads (testes in male and ovaries in female). The organs of the system secrete one or more hormones which aid in the proper metabolic function of a target organ, for example, ADH, an antidiuretic hormone, sometimes called vasopressin secretes from the posterior pituitary gland to promote the reabsorption of water by the kidney. This is thought to be regulated by the hypothalamus.

The hyopthalamus is believed to regulate the secretion of the antidiuretic hormone ADH. When specialized cells of the hypothalamus sense that the blood lacks sufficient water, ADH is released by the hyopthalamus nerve fibers for secretion by the posterior pituitary. ADH travels in the bloodstream to its target organ, the kidney. There it promotes the reabsorption of water so that the blood becomes more dilute. As the blood becomes more dilute because of the reabsorbed water, this fact is sensed in the hypothalamus, and production of the ADH ceases.

In some instances, the target organ of one endocrine gland is a second gland in the same system. For example, the trophic hormones of the anterior pituitary, e.g. luteinizing hormone (LH), stimulate the gonads to produce androgens, such as testosterone, estrogen and progesterone.

Growth hormone (GH) produced in the anterior pituitary dramatically affects the phenotype of mammals since it determines the size of the individual. It causes protein accumulation in almost all of the cells of the body. In response to GH, all tissues of the body including bones grow. The bones continue to grow during childhood as long as a small area of cartilage remains at both ends. Growth ceases when this area of cartilage is replaced by bone. The long bones have completed their growth at the onset of adulthood.

The amount of GH produced during the early prepubertal years determines the size of the individual. If the production is too low, or completely absent, the person will become a midget. If too much GH is produced the person will become a giant.

If the production of GH increases in the,adult only the bones of the jaw, eyebrows, nose, fingers and toes can respond. When these begin to grow, the person takes on an unusual appearance characterized by very large fingers and toes. This condition is one result of hyperpituitarism. It is specifically identified as acromegaly. It is believed that the biblical figure Goliath had acromegaly. Other such conditions are known and recognized.

In instances where there is an overproduction of hormones which may be manifested by undesirable weight gain or increase in growth rate, or where there is an undesirable response to normal production of hormones, it would be useful to suppress the production of the offending hormone or hormones. Such a method has now been discovered.

It has been found that it is possible to suppress the endocrine system of mammals by treatment with a selected metalloporphyrin thereby making it possible to treat various human afflictions associated with overactivity of one or more of the glands of the endocrine system. More specifically, it has been observed that the administration to mammals of CoPP strongly stimulates heme oxygenase activity while at the same time suppressing ALA synthetase activity and markedly suppressing the endocrine system.

A particularly valuable aspect of this invention is the ability of CoPP to limit the production of the gonadal androgenic and estrogenic hormones such as testosterone and estrogen, and to limit the production of the thyroid hormones.

The gonadal hormones have anabolic activity. They and their synthetic analogs are employed to promote growth. Overproduction of the anabolic hormones leads to an increased growth rate associated with increased food intake. It has been observed that one of the immediate effects of the administration of CoPP in mammals is a prompt decrease in appetite and subsequent growth rate. This continues during the period when the concentration of gonadal hormones is below that of untreated mammals. Suprisingly, the decreased growth rate continues long after the gonadal level has returned to normal. In rats, as will be explained below, the low testosterone level continues for about 48 days, but the decreased growth rate continues for at least 90 to 100 days. In all other respects the animal appears to be normal. The period of the decreased testosterone level may be controlled for selected periods of time by selection of the amount of CoPP administered.

The decreased growth rate observed in animals can be maintained beyond the 90 days or other originally selected period by further administration of CoPP, typically at a lower dosage level. Thus CoPP can be used in mammals to attain and maintain decreased growth rates, thereby assisting in weight control.

Similar results are achieved with CoMP.

As shown below, CoPP administered to rats results in a very marked depletion in plasma of the thyroid hormones, $T_3$ and $T_4$ as well as lower than expected levels of the pituitary derived trophic hormone, TSH. Additionally, in such treated animals there is a loss of gonadal hormones such as testosterone and estrogen from plasma and a concurrently lower than normal level of the pituitary derived trophic hormone, LH. It has also been observed on the hypothalamus - pituitary - adrenal axis that there is a suppression of cortisol in plasma.

These findings indicate that the peripheral endocrine system as represented by the thyroid, gonads and adrenals is suppressed and that the result is related to suppression of the synthesis or release of pituitary hormones. Microscopic examination on the pituitary indicate that the thyroid releasing cells (TSH) are markedly smaller in the CoPP treated animals than in normal control animals.

The figures show the results of studies conducted with rats, and clearly illustrate the activity of CoPP to suppress the activity of the endocrine system. Similar results are obtained with CoMP.

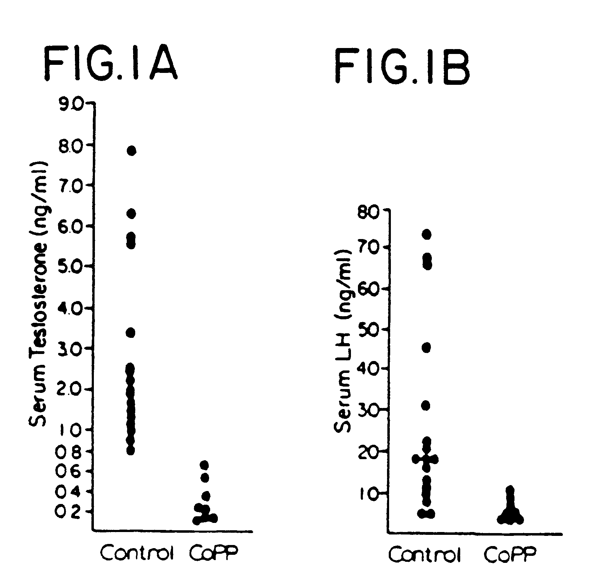

Figure 1, panels A and B, depicts graphically the results of experiments showing that CoPP suppresses the endocrine system in rats. Specifically, in this example, it is shown that the aspect of the endocrine system involving the pituitary-testicular axis is suppressed. Panel A of Fig. 1 shows serum testosterone levels in rats which received 2 doses of CoPP with hormone determinations being made 7 days after the initial injection. As can be seen in the control column on the left, serum testosterone levels before treatment with CoPP were within the range expected in this animal species. In the group of data shown in the right column of this panel (marked CoPP), the levels of serum testosterone after CoPP administration are all below 0.8 ng/ml which is an extraordinary drop in the serum level of this male sex hormone. In panel B on the right, data showing serum LH levels, which controls the synthesis and release of testosterone from the gonads and which itself is produced and released by the pituitary under hypothalamic stimulation, are shown. The control group shows the range of normal levels of serum LH which are characteristic of this animal species. The data for CoPP shown at the right of this panel indicate that there was no compensatory increase in serum LH release from the pituitary as would normally be expected despite the profound decline in serum testosterone levels shown in the left panel of this figure.

In these studies, the dosage level of CoPP was 50 µmol/kg of body weight. The number of animals is indicated by the number of dots.

There is known to be a feed-back regulatory endocrine mechanism in which the peripheral and central endocrine glands, i.e. pituitary - gonadal axis in this case, act in concert to keep hormone levels at an appropriate constant level of equilibrium. Thus, if serum testosterone levels markedly decline, there should be a striking increase in serum LH levels to restore serum testosterone levels to normal. In contrast to that expected increase, the CoPP treated animals showed no such compensatory increase whatsoever, as can be clearly seen,from Fig. 1 B. This indicates that the compensatory mechanism for stimulating an enhanced output of serum testosterone had been impaired by the CoPP and that for reasons which are not clear, the pituitary was unable to release LH which would be required to compensate the disequilibrium in testosterone production produced by CoPP. Thus the decrease in serum testosterone levels in these animals was not compensated by a rebound increase in serum LH which would have brought the serum sex hormone levels back to normal. This indicates a profound decline in endocrine function at both the central and peripheral levels. It strongly suggests that the synthetic metalloporphyrin operates at a level even higher than the pituitary gland and specifically at some point in the axis between the pituitary-hypothamalus from which releasing hormones (i.e. LH-releasing hormone etc.) are transmitted to the pituitary where they then stimulate the release of such trophic hormones as, in this case, LH.

The results with CoMP are similar,

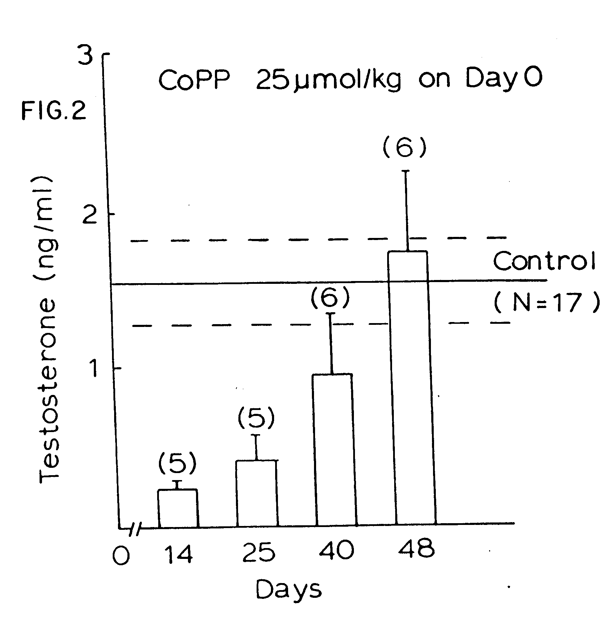

Figure 2 shows the duration of the suppression of serum testosterone levels that can be elicited by a single dose of 25 µmol/kg of body weight of CoPP. As indicated, the bars show the level of serum testosterone which on days 14-40 are markedly below normal levels as in consistence with the data in Figure 1. Hormone levels gradually revert towards normal levels - but do not enter the normal range, until day 48. Thus a single dose of CoPP is capable of profoundly suppressing the synthesis and release of gonadal sex and growth hormone testosterone and the synthesis or release of the trophic hormone for gondal hormone production (LH). This effect can last for 40-48 days after a single injection of either of the metalloporphyrins of this invention. This ability transiently to suppress such an important endocrine complex as the hyopthalamus-pituitary-gonadal axis is a novel biological action effected by administration of the herein disclosed metalloporphyrins and clearly shows the utility of these products for treatment of disorders that are provoked by excessive levels of the gonadal hormones. In other studies the suppression depicted here over a term of 48 days, has been extended for as long as 9 months by repeated administration of CoPP at periodic intervals. This clearly indicates that periodic administration of this synthetic metalloporphyrin will provide a useful means for suppressing endocrine function for as long as the compound is administered.

In Figure 2 the number above each bar is the number of animals tested. The dotted, horizontal lines represent the range observed for levels of testosterone in the control animals.

The suppression of the endocrine system resulting in markedly suppressed production of gonadal and thyroid hormones by CoPP and CoMP is reversible. Once the administration of the selected compound ceases, there is a gradual and complete return to normal of the endocrine system. This characteristic of the compounds is of great value in clinical situations in which the degree to which a disorder is responsive to excessive levels of a particular endocrine secretion is not known. It can be determined by judicious use - to suppress endocrine function - of an appropriate dose of CoPP or CoMP, permitting recovery to normal when the effect of the compound has receded. However, although the endocrine system by standard measures returns to normal, the growth rate remains at a reduced level for many more weeks.

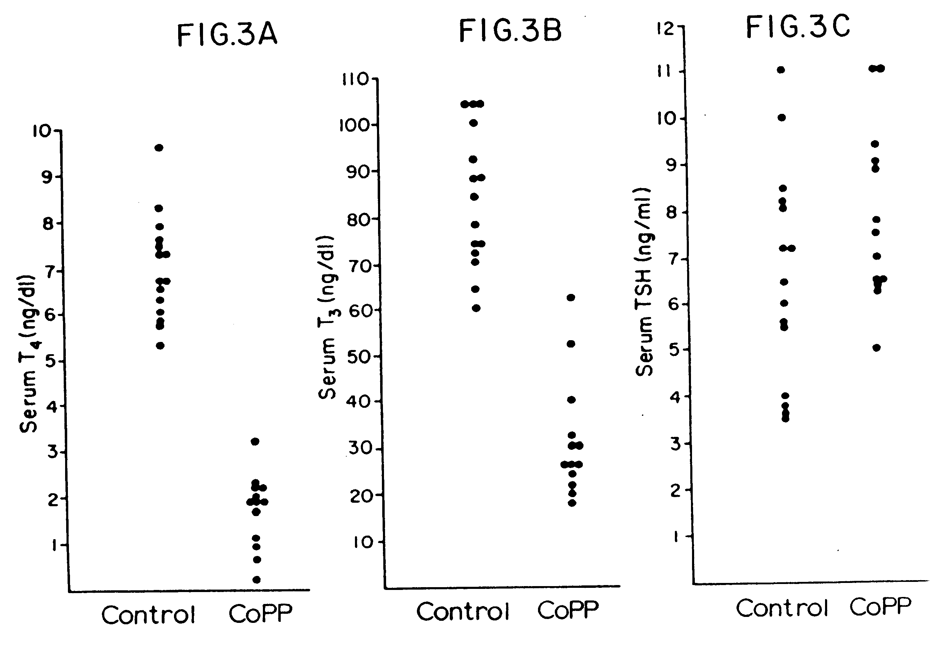

Figure 3 shows comparable data for the pituitary-thyroid axis. Panel A of the figure shows serum $T_4$ (thyroxin) levels in animals and the range over which they fall normally. The column marked CoPP in this panel shows the markedly diminished levels of serum $T_4$ which are produced by treatment of the animals with CoPP (2 to 50 $\mu$ mol/kg b.w.) in the same manner and with the same dosages indicated for the previous studies with the sex hormone, testosterone. Panel B shows a similar decrease in levels of the second major thyroid hormone, $T_3$, its levels in plasma in control animals, and the comparable depleting effect on the plasma levels of this hormone produced by CoPP. Finally, Panel C shows the serum levels of the trophic hormone for the thyroid gland, i.e. thyroid stimulating hormone (TSH), in both control and CoPP-treated animals. In animals including man, when thyroid levels are markedly depleted, as shown in the first two panels of this figure, serum TSH levels will rebound to levels far greater than are seen in control circumstances. However, as panel C indicates, there was no such rebound in the CoPP-treated animals, indicating that the pituitary gland in these animals was incapable of responding normally to the decreased plasma levels of thyroid hormones ($T_4$ and $T_3$) that were produced by CoPP.

Similar results are observed with CoMP.

These findings together with those in Figures 1 and 2 dealing with the pituitary-gonadal axis clearly indicate a hypothalmic effect of the synthetic metalloporphyrins of this invention. This effect could be mediated through an action of the compound on the releasing hormones for the trophic hormones (LH or TSH) which control the peripheral synthesis and secretion of thyroid and gonadal hormones, or by some other mechanism as yet undefined. Thus, in these two major endocrine axes, the synthetic metalloporphyrins CoPP and CoMP exert a profound suppressive effect following administration in relatively small amounts of the selected compound.

It is clear from the results of these studies involving the pituitary-thyroid axis that administration of CoPP is useful to treat mammalian afflictions associated with hyperthyroidism.

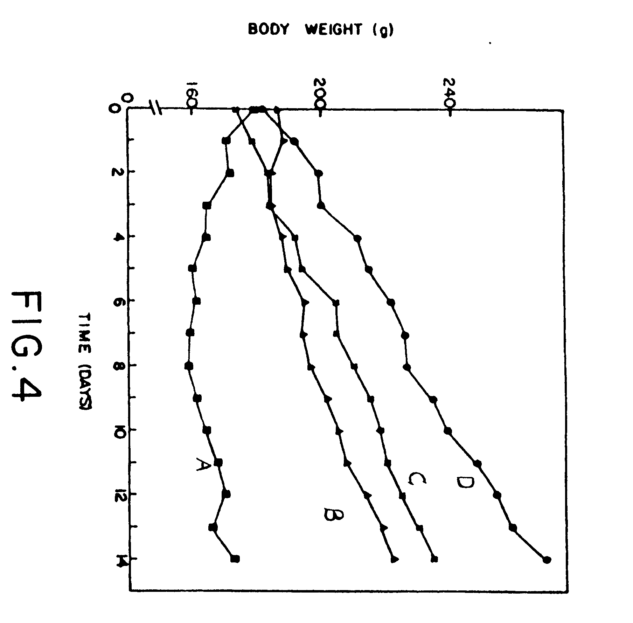

Figure 4 shows the observed weight loss after-a single dose of CoPP treatment of male Sprague-Dawley rats (140-160g).

The parenteral solutions used in this study and other studies reported herein can be prepared by dissolving CoPP or CoMP in a small volume of 0.1M NaOH (0.1ml/ml of the metalloporphyrin solution). The CoPP and CoMP were purchased from Porphyrin Products (Logan, UT.). The pH of the solution was adjusted to 7.4 with 1M HCl, and the final volume was adjusted by the addition of 0.9% NaCl solution. Treatment was by subcutaneous injection.

In the study, the rats were treated on day zero with the following CoPP solutions:

50 $\mu$mol/kg bw     A
2 5 $\mu$mol/kg bw     B
5 $\mu$mol/kg bw     C
saline     D

Each point in the figure represents the mean body weight of 6 animals.

It will be seen that after an initial cessation of, or decline in, rate of growth, rats treated with 5-25 $\mu$mol/kg b.w. gained at a rate parallel to, but below that of saline treated controls. Rats treated with 50 $\mu$mol/kg b.w. lost weight immediately and had not returned to their starting weight even after 14 days. However by day 14 they had started to gain weight at a rate parallel to the controls.

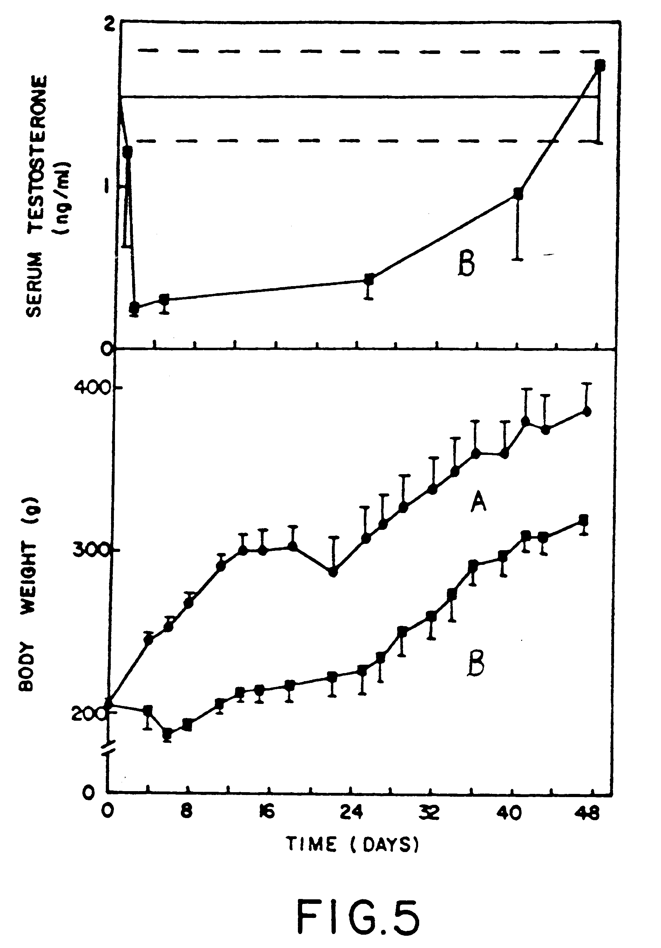

Figure 5 shows a relationship between testosterone concentration and weight loss. The lower panel, shows the daily weight $\pm$SEM of 6 rats treated with saline, A, or CoPP at 25 $\mu$g/kg b.w. At the times indicated tail blood was collected and serum testosterone concentration measured (upper panel). The value $\pm$SEM of 6 treated rats B are shown in the upper panel.

In this study serum testosterone levels declined by about 90% within 4 days. Weight gain remained constant during this 4 day period, dropped slightly in the ensuing week, and resumed in succeeding weeks, paralleling controls from about day 20 to day 48. Untreated animals gained weight steadily throughout the study period. Within the time period from 4 to 25 days, serum testosterone levels remained at markedly low levels and thereafter returned toward normal which they reached after about 40 to 48 days.

Despite the fact that treated animals reverted to a rate of weight gain paralleling that of the controls, even when their serum testosterone levels had normalized, their total body weights remained consistently about 25% lower than those of untreated animals. It was noted that this disparity continued for as long as 90 to 100 days. Similar results are observed in female rats by studying the relationship between estrogen levels and weight gain.

It appears that the administration of CoPP or CoMP to mammals may have two effects. These are:

1. A transient decrease in gonadal hormone production with a concommitant decrease in anabolic or growth promoting activity.

2. A long term appetite suppressing effect possibly by action of the compounds on some appetite regulatory site in the hypothalamus.

It should be noted that endocrine studies in animals have served as valid and art recognized models of the regulatory mechanisms by which the endocrine control systems in humans operate. Thus, for example, the seminal work by Huggins on the role of male sex hormone in the pathogenesis of prostatic hypertrophy and the effects thereon of castration (removal of the male sex hormone, testosterone, by removing the testes) with resulting remission of the hypertrophic prostate condition was done in dogs. Similarly, the elucidation of the principal regulatory mechanisms for thyroid hormone have been carried out in rats and other animals, and the results observed have proved to be reliable indicators of analogous results in humans thus permitting accurate predictions of therapeutic results to be achieved by administration to humans of agents tested in animals. Thus, these animal models of endocrine suppression by the CoPP have full relevance to the circumstances existing in humans and clearly indicate that in selected doses the synthetic metalloporphyrins CoPP or CoMP will suppress the endocrine system in humans and other mammals.

The newly discovered ability markedly but transiently to suppress the endocrine system of mammals as described and illustrated herein has very important clinical consequences because there are circumstances in which the extent to which a certain condition, for example a hypertrophic growth condition in the prostate or thyroid is hormone-sensitive is now known. In the past, this problem has often forced the physician to extirpative surgery (castration, thyroidectomy or hypophysectomy) As a result of this discovery, it is now very simple to administer small doses of CoPP, suppress the endocrine system (with selective replacement of hormones not suspected of being provocative agents) and determine the effect of the suppression on the course of the condition in question. Following the obtaining of such information, therapy is then continued with the selected metalloporphyrin to maintain suppression of the endocrine system or selective surgical alteration of,the appropriate endocrine gland could be carried out if warranted.

The selected compounds of the invention will normally be administered parenterally, i.e., intraveneously, subcutaneously or intramuscularly in sterile, parenteral, isotonic solutions. For such solutions any of a wide variety of pharmaceutically acceptable carriers currently in use for the preparation of parenteral compositions may be employed. The solutions may be buffered for example with a phosphate buffer to a pH of about 7 to 8, preferably 7.4 to 7.5, and contain solutes such as saline or glucose. The solutions may also contain a polyhydroxy alcohol such as ethylene or propylene glycol. The active compounds may also be administered in solution or suspension in a sterile, inert oil such as sesame or sunflower oil. A typical dosage regimen will be two separate administrations per week at a dosage rate of 1 to 50 $\mu$mol/kg of body weight. The physician or veterinarian will determine the specific dosage, and it will depend upon such well understood factors as the condition under treatment, age, weight and general health of the patient.

Typically, an isotonic solution can be prepared by dissolving the selected amount of CoPP or CoMP in 0.1M aqueous sodium hydroxide solution, adjusting to the selected pH with 1M hydrochloric acid, and making up to volume with 0.9 aqueous sodium chloride solution. The concentration of active agent in the parenteral composition may be from about 1 to 25 mg/ml.

The administration of the active agent appears to suppress the complete endocrine system but is particularly valuable for limiting the production of gonadal and thyroid hormones and for controlling weight. Therefore, as suggested above, if the active agent is administered to control one endocrine function, e.g. secretion of thyroid hormone, there will be a concurrent treatment with other supplemental hormones, all of which are available commercially, to compensate for the suppression of other endocrine functions

Except for growth rate the suppressive effect of CoPP and CoMP recedes with time and the subjects revert to their normal state except as to growth rate and ultimate body weight after variable periods of time depending on the dosage of the metalloporphyrin. Thus the treatment can be continued for selected periods to correct or alleviate a specific condition. It may be interrupted after a first course of treatment, or it may be continued for differing periods of time at different levels.

Inorganic cobalt, iron protoporphyrin (heme), tin protoporphyrin and other metalloporphyrins do not have the effect of CoPP and CoMP. It presently appears that the requirements for endocrine system suppression are that the metalloporphyrin be capable of stimulating for extended periods of time the catalytic activity of heme oxygenase 5 to 15 fold in the liver and perhaps pituitary glands and hypothalmus while simultaneously suppressing ALA-synthetase and cytochrome P-450 activity in these organs. These enzymes are the rate limiting enzymes of heme degradation and heme synthesis respectively. They may be involved in the mechanism by which the active agent of the invention suppresses the endocrine system.

## Claims

1. The use of cobalt protoporphyrin and/or cobalt mesoporphyrin for the production of a pharmaceutical composition for suppressing the formation of hormones of the endocrinic system in mammals.

2. Use according to claim 1, wherein the hormones of the endocrinic system are gonadal hormones.

3. Use according to claim 1, wherein the hormones of the endocrinic system are thyroid hormones.

4. Use according to claim 1, wherein the pharmaceutical composition is for parenteral administration.

5. Use of cobalt protoporphyrin and/or cobalt mesoporphyrin for the production of a pharmaceutical composition for parenteral administration for controlling weight gain in mammals.

## Patentansprüche

1. Die Verwendung von Kobaltprotoporphyrin und/oder Kobaltmesoporphyrin zur Herstellung einer pharmazeutischen Zusammensetzung für die Suppression der Bildung von Hormonen des endokrinen Systems bei Säugetieren bzw. dem Menschen.

2. Verwendung gemäß Anspruch 1, bei der die Hormone des endokrinen Systems Gonadenhormone sind.

3. Verwendung gemäß Anspruch 1, bei der die Hormone des endokrinen Systems Schilddrüsenhormone sind.

4. Verwendung gemäß Anspruch 1, bei der die pharmazeutische Zusammensetzung zur parenteralen Verabreichung bestimmt ist.

5. Verwendung von Kobaltprotoporphyrin und/oder Kobaltmesoporphyrin zur Herstellung einer pharmazeutischen Zusammensetzung für die parenterale Verabreichung zur Regelung der Gewichtszunahme bei einem Säugetier bzw. dem Menschen.

## Revendications

1. Utilisation de protoporphyrine de cobalt et/ou de mésoporphyrine de cobalt pour produire une composition pharmaceutique pour supprimer la formation d'hormones du système endocrinien chez des mammifères.

2. Utilisation selon la revendication 1, dans laquelle les hormones du système endocrinien sont des hormones gonadiques.

3. Utilisation selon la revendication 1, dans laquelle les hormones du système endocrinien sont des hormones thyroïdiennes.

4. Utilisation selon la revendication 1, dans laquelle la composition pharmaceutique est destinée à une administration parentérale.

5. Utilisation de protoporphyrine de cobalt et/ou de mésoporphyrine de cobalt pour la production d'une composition pharmaceutique pour une administration parentérale pour commander le gain de poids chez des mammifères.

FIG.IA

FIG.IB

FIG.2

CoPP 25μmol/kg on Day 0

Control

( N=17 )

(6)

(6)

(5)

(5)

Testosterone (ng/ml)

Days

0   14   25   40   48

FIG.3A     FIG.3B     FIG.3C

EP 0 259 353 B1

FIG.4

FIG.5